# EUROPÄISCHE PATENTSCHRIFT

(11) **EP 2 515 756 B1**
(45) Veröffentlichungstag und Bekanntmachung des Hinweises auf die Patenterteilung: **10.12.2014**
(21) Anmeldenummer: 10801578.5
(22) Anmeldetag: 21.12.2010
(51) Int. Cl.: G01N 21/39, A61B 5/083, G01N 21/3504

(54) **MESSVORRICHTUNG UND VERFAHREN ZUR UNTERSUCHUNG EINES PROBEGASES MITTELS INFRAROT-ABSORPTIONSSPEKTROSKOPIE**
MEASURING DEVICE AND METHOD FOR ANALYSING A TEST GAS BY MEANS OF INFRARED ABSORPTION SPECTROSCOPY
DISPOSITIF DE MESURE ET PROCÉDÉ POUR ANALYSER UN GAZ DE MESURE PAR SPECTROSCOPIE D'ABSORPTION INFRAROUGE

(30) Priorität: 24.12.2009 DE 102009055320
(43) Veröffentlichungstag der Anmeldung: 31.10.2012
(60) Teilanmeldung: 13159182.8
(73) Patentinhaber: Humedics GmbH, 10625 Berlin (DE)
(72) Erfinder: HEYNE, Karsten, 14979 Großbeeren (DE); RUBIN, Tom, 12163 Berlin (DE)
(74) Vertreter: Sokolowski, Fabian
(86) Internationale Anmeldenummer: PCT/EP2010/070407
(87) Internationale Veröffentlichungsnummer: WO 2011/076803

(56) Entgegenhaltungen:
- WO-A1-99/61895
- WO-A1-2009/101374
- GB-A- 2 312 743
- JP-A- 5 340 872
- US-A1- 2004 211 905
- US-B1- 6 216 692
- anonymous: "Atmung", Wikipedia, 18. April 2011 (2011-04-18), Seiten 1-9, XP7918339, Gefunden im Internet: URL:http://de.wikipedia.org/wiki/Atmung [gefunden am 2011-04-18]
- WEIDMANN D ET AL: "Development of a compact quantum cascade laser spectrometer for field measurements of CO2 isotopes", APPLIED PHYSICS B ; LASERS AND OPTICS, SPRINGER, BERLIN, DE, Bd. 80, Nr. 2, 1. Februar 2005 (2005-02-01), Seiten 255-260, XP019337285, ISSN: 1432-0649, DOI: DOI:10.1007/S00340-004-1639-7

## Beschreibung

Die Erfindung betrifft eine Messvorrichtung zur Untersuchung eines Probegases mittels Infrarot-Absorptionsspektroskopie gemäß dem Oberbegriff des Anspruchs 1 sowie ein entsprechendes Verfahren.

Seit Jahrhunderten ist bekannt, dass der Atemgeruch ein Indikator für eine mögliche Erkrankung sein kann - das prominenteste Beispiel ist der süßlich-fruchtige, durch Aceton verursachte Geruch bei Diabetes mellitus Typ 1. Selbst der Atem gesunder Menschen enthält mehrere hundert flüchtige chemische Verbindungen, sogenannte "volatile organic compounds" (VOCs), in geringer Konzentration (ppm- bis ppt-Bereich). Einige davon spielen bei physiologischen bzw. pathophysiologischen Prozessen eine wichtige Rolle. Liegt eine Erkrankung vor, steigt die Konzentration von bestimmten Spurengasen im Atem an. Bei manchen Erkrankungen lassen sich auch Gase nachweisen, die im gesunden Organismus nicht vorkommen. Somit bietet die Atemgasanalyse ein großes Potenzial für die klinische Diagnostik und Therapieüberwachung. Allerdings ist die Spurengas-Konzentration häufig so gering, dass sie mit den verfügbaren gasanalytischen Verfahren nicht ausreichend genau gemessen werden kann.

Es existieren hochempfindliche Nachweisverfahren, wie z.B. die Massenspektroskopie oder die FTIR-Spektroskopie in Mulitpass-Probenzellen. Allerdings sind solche Nachweisgeräte nicht direkt am Patienten einsetzbar und somit für den klinischen Alltag bedeutungslos. Das liegt auch daran, dass die Auswertung mehrere Tage dauert und durch den Transport der Probe unkalkulierbare Fehlerquellen auftreten. Mobile Aufbauten im Bereich der Infrarotabsorptionsspektroskopie mit Diodenlasern (z.B. Bleisalzlasern) als Lichtquellen sind ebenfalls seit mehreren Jahrzehnten im Einsatz, erreichten aber bis jetzt nicht die benötigte Stabilität über einen längeren Zeitraum für den empfindlichen Nachweis von Gasen, so dass auch hier der Einsatz auf die medizinische Grundlagenforschung beschränkt blieb.

Eine alternative Methode ist das sogenannte NDIRS-Verfahren (NDIRS - Nicht-Dispersive IR-Spektroskopie). Es weist Dichteschwankungen im Probegas nach, die durch Absorption von Infrarotlicht ausgelöst werden. Dieses Nachweisverfahren ist empfindlich und kann alle zweieinhalb Minuten eine Messung durchführen. Allerdings werden die Messergebnisse durch andere Gase wie z.B. Sauerstoff verfälscht, so dass diese Methode nur eingeschränkt im klinischen Alltag eingesetzt werden kann.

Eine weitere Methode wird von dem Unternehmen Oridion Systems Ltd. unter der Bezeichnung BreathID® eingesetzt. Hier wird als Lichtquelle eine CO₂-Drucklampe verwendet. Allerdings ist diese Methode in ihrer Empfindlichkeit und Schnelligkeit durch auftretende Linienbreitenschwankungen (in der Lampe und im Probengas), geringe Lichtintensitäten und spektrale Fluktuationen stark eingeschränkt und liefert dadurch keine hochempfindlichen Messergebnisse in kurzer Zeit. Die NDIRS Methode und die Methode von Oridion Systems Ltd. eignen sich z.B. gut zum Nachweis des Bakteriums *Helicobacter pylori* im Patientenmagen. Die Anwesenheit des Bakteriums wird über einen erhöhten ¹³CO₂-Gehalt in der Ausatemluft nach Verabreichung eines ¹³C-markierten Diagnostikum qualitativ nachgewiesen.

Qualitative Testverfahren werden bedeutungslos, wenn der Test im gleichen Preissegment wie die Behandlung liegt. Eine weitere Strategie, um den einfachen und schnellen Nachweis von flüchtige chemische Verbindungen zu gewährleisten, ist der Einsatz von oberflächensensitiven Mikrochips, die spezielle Spurengase aus der Atemluft selektieren und binden. Damit ist ein empfindlicher Nachweis der flüchtige chemische Verbindungen möglich und die qualitative Entscheidung, ob der Patient erkrankt ist oder nicht, kann getroffen werden.

Der reine Nachweis einer Erkrankung ist aufschlussreich, gibt allerdings noch keine Auskunft über die geeignete Therapie. Daher liegt die Zukunft der Atemgasanalyse in der quantitativen Bestimmung des Erkrankungsgrades, die dem Arzt direkte Entscheidungshilfen für die Therapie an die Hand gibt. Wenn solche Tests einfach und schnell durchführbar sind und die Ergebnisse sofort für den Arzt in verständlicher Form vorliegen, kann sich der Test im klinischen Alltag durchsetzen.

Die Anforderungen an quantitative Atemgastests sind hoch: Zur eindeutigen Identifizierung der Spurengase ist eine hohe Selektivität und Nachweisempfindlichkeit erforderlich, da die Konzentration meist im ppm- bis ppb-Bereich liegt. Die exakte quantitative Bestimmung der Spurengasmenge muss gewährleistet sein. Außerdem sollten die Messungen online und in Echtzeit erfolgen, um eine aufwendige und fehleranfällige Probensammlung (z. B. in Beuteln oder im Seitenstrom) zu vermeiden. Für eine praktikable und wirtschaftliche Nutzung sind leichte Bedienbarkeit, Kompaktheit, Robustheit, geringer Wartungsaufwand und/oder ein günstiges Kosten-Nutzen-Verhältnis zu fordern. Diese hohen und vielfältigen Anforderungen können derzeit von keinem gasanalytischen Verfahren vollständig erfüllt werden.

Die ausgeatmete Luft von Menschen weist einen Kohlendioxidvolumenanteil von 2% bis 4% auf und wird in 10 bis 20 Atemzügen pro Minute, bei Kleinkindern und Neugeborenen sogar in 25 bis 50 Atemzügen pro Minute ausgeatmet. Die Atemdruck des Menschen beträgt etwa 50mbar bis maximal 160mbar, bei einem Volumen von etwa 0,5 Litern. Von der Atemluft gelangen nur etwa 70% in die Lunge, so dass auch nur in etwa 70% des Gasvolumens ein deutlich erhöhter Kohlendioxidanteil vorliegt. In dem restlichen Gasvolumen - dem Totraumvolumen - kann die Kohlendioxidkonzentration bis auf die Konzentration der Umgebungsluft von etwa 0,04% sinken. Dies führt dazu, dass die Kohlendioxidkonzentration der Atemluft um zwei Größenordnungen von 0,04% bis 4% schwanken kann. Kohlendioxidkonzentration von über 5% sind toxisch und können z.B. zu Kopfschmerzen und Krämpfen führen.

Die produzierte Kohlendioxidmenge hängt von dem individuellen Stoffwechsel jedes einzelnen Menschen ab. Es werden verschiedene Näherungsverfahren verwendet um die Kohlendioxidproduktion eines Menschen abzuschätzen. Die Größen, die dort eingehen, sind z.B. Gewicht und Körperoberfläche. Die Körperoberfläche wird meistens wiederum mit dem Gewicht und der Körpergröße abgeschätzt, so dass in der Medizin oftmals mit nur mäßig genauen Größen gerechnet wird, was eine quantitative Ergebnisauswertung stark einschränkt oder sogar unmöglich macht.

Zur direkten quantitativen Bestimmung von Stoffwechselvorgängen oder Metabolisierungen ist es erforderlich, die Dynamik des Prozesses zeitaufgelöst zu verfolgen, am besten in Echtzeit. Weist die Metabolisierungsdynamik eine Kinetik auf, die durch eine Differentialgleichung 1. Ordnung modelliert werden kann (Dynamik 1. Ordnung), so können durch Lösung der Differentialgleichung oder durch Anpassen einer Exponentialfunktion: y(t)=A*exp(-t/tau) das Maximum der Kinetik A und die Zeitkonstante tau bestimmt werden. Aus den Größen A und tau können dann quantitativ metabolische Parameter bestimmt werden. Die Auslösung der Metabolisierungsdynamik wird bestenfalls durch eine zeitlich kurze Initiation erreicht, z.B. die iv-Verabreichung eines Diagnostikums oder die Freisetzung eines Diagnostikums durch Belichtung/Bestrahlung.

Dauert die Freisetzung oder der Start der Dynamik länger als der Anstieg tau oder als ein Atemzug, so muss die Dynamik der Freisetzung gesondert bestimmt werden und von der Metabolisierungsdynamik entfaltet werden. Ein Beispiel für einen schnellen Metabolisierungsstart ist die iv-Verabreichung des Diagnostikums 13C-Methacetin im Bolus. Es wird mit dem Blut (ca 60 Herzschläge pro Minute) im Körper verteilt und gelangt in etwa einer Sekunde zur Leber, wo es metabolisiert wird zu Paracetamol und 13CO2. Der Start der Dynamik ist viel schneller als der Atemrhythmus und führt so zu einer Dynamik 1. Ordnung, die direkt ausgewertet werden kann. Wird das 13C-Methacetin allerdings oral verabreicht, führt die Adsorption im Magen zu einer Faltung der Dynamik mit der Magenadsorptionsdynamik, die die Dynamik signifikant verfälscht.

Um die Metabolisierungsdynamik in Echtzeit zu verfolgen, sollte jeder Atemzug mit sehr hoher Empfindlichkeit gemessen werden. Das bedeutet, dass die Atemluft in der Messkammer schnell ausgetauscht werden muss und dass in weniger als zwei Sekunden eine komplette Auswertung des Atemzuges erfolgt sein muss.

Ein Analyseverfahren, welches eine quantitative Bestimmung der Leberfunktion ermöglicht, ist in der WO 2007/000145 A2 beschrieben. Das Verfahren basiert auf einer Substratanflutung des zu metabolisierenden Substrates in der Leber und der Bestimmung der maximalen Umsatzgeschwindigkeit des Substrates, die Aussagen über die Leberfunktionskapazität eines Patienten ermöglicht.

WO 99/61895 und GB 2312743 A offenbaren Systeme zur Überwachung der ¹²CO₂ und ¹³CO₂ Konzentration in Atemluft, wobei jeweils ein Diodenlaser über die Absorptionskennlinien der Isotope durchgestimmt wird, um das Absorptionsspektrum zu detektieren.

Aus der WO 2007/107 366 A1 ist eine gattungsgemäße Vorrichtung zur spektroskopischen Analyse eines Gases bekannt, bei der eine Messkammer kontinuierlich mit einem Probegas durchströmt werden kann.

Der vorliegenden Erfindung liegt die Aufgabe zugrunde, die aus der WO 2007/107 366 A1 bekannte Messvorrichtung und das dort verwendete Messverfahren mit dem Ziel, Messungen in Echtzeit durchführen zu können, zu verbessern.

Diese Aufgabe wird erfindungsgemäß durch eine Messvorrichtung mit den Merkmalen des Anspruchs 1 und ein Verfahren mit den Merkmalen des Anspruchs 13 gelöst. Ausgestaltungen der Erfindung sind in den Unteransprüchen angegeben.

Danach sieht die erfindungsgemäße Lösung vor, einen schmalbandig emittierenden Laser einzusetzen. Als schmalbandig emittierender Laser wird ein Laser angesehen, dessen Linienbreite derart gewählt ist, dass sie kleiner oder gleich der Breite der zu messenden Absorptionslinie des Probegases ist. Weiter ist vorgesehen, dass die Laserfrequenz periodisch innerhalb eines definierten Spektralbereichs variiert wird, wobei die Laserfrequenz und deren Variation derart gewählt sind, dass mindestens eine zu messende Absorptionslinie des Probegases in dem definierten Spektralbereich liegt. Die periodische Variation der Laserfrequenz (auch als Durchstimmbarkeit bezeichnet) geht dabei einher mit einem definierten Spektralbereich, der während jeder Periode dieser Frequenzvariation ausgemessen wird. In diesem Spektralbereich liegt mindestens eine zu messende Absorptionslinie.

Es ist erfindungsgemäß weiter vorgesehen, dass die Detektionsvorrichtung dazu ausgebildet und eingerichtet ist, das vom Laser emittierte und die Messkammer durchstrahlte Licht derart zeitaufgelöst zu erfassen, dass die Lichtabsorption innerhalb des definierten Spektralbereiches frequenzaufgelöst erfasst werden kann. Dabei führt die Detektionsvorrichtung eine einzelne Absorptionsmessung innerhalb von 10⁻⁵ Sekunden oder schneller durch, insbesondere innerhalb von 10⁻⁶ Sekunden oder schneller. Durch die schnelle Messung kann ein Spektralbereich, der durch die Variation der Laserfrequenz ausgemessen wird, frequenzaufgelöst erfasst werden. Der ausgemessene Spektralbereich wird dabei mit einer Mehrzahl von Messpunkten, die jeweils einer Absorptionsmessung entsprechen, beispielsweise mit 20, 100, 500 oder 1000 Messpunkten ausgemessen.

Die hohe Zeitauflösung der Absorptionsmessungen erlaubt es, einen Spektralbereich, der durch die Variation der Laserfrequenz definiert wird und in dem mindestens eine zu messende Absorptionslinie liegt, frequenzaufgelöst mit einer hohen Punktdichte von Einzelmessungen innerhalb des Spektralbereichs zu erfassen. Dies ist mit mehreren Vorteilen verbunden.

So geht die hohe Zeitauflösung und die dadurch erreichbare hohe Punktdichte der Messwerte eines während einer Frequenzvariation ausgemessenen Spektralbereichs mit einer hohen Messgenauigkeit einher. Diese wird weiter erhöht, wenn eine Mittelung über mehrere hintereinander erfasste Spektren erfolgt, wie es in einer Ausführungsvariante vorgesehen ist.

Die hohe Zeitauflösung, die hohe spektrale Auflösung (erreicht durch eine hohe Punktdichte der Einzelmessungen) und eine hohe Empfindlichkeit erlauben es, Absorptionslinien mit einer Sensitivität im ppb-Bereich zu messen. Eine solche Sensitivität ist essentiell notwendig, um beispielsweise einen Einsatz der Messvorrichtung für den quantitativen Nachweis für metabolisierte Substrate zu gewährleisten.

Die Messvorrichtung ist darüber hinaus insbesondere dazu geeignet und einrichtbar, als Probegas das Atemgas eines Menschen oder Tieres zu messen, wobei das Atemgas allein durch die Atmung des Menschen oder des Tieres in der Messkammer austauscht und der Atemwiderstand der Messvorrichtung bei weniger als 60 mbar, insbesondere weniger als 50 mbar und ganz besonders weniger als 40 mbar liegt. Das heißt, es sind keine Pumpen oder anderen Vorrichtungen notwendig, um das Probegas durch die Messvorrichtung hindurch zu befördern. Mit anderen Worten ausgedrückt, beträgt der von der Messvorrichtung aufgebaute Gegendruck weniger als 60 mbar, insbesondere weniger als 50 mbar und ganz besonders weniger als 40 mbar. Ein solch niedriger Gegendruck kann ohne technische Hilfsmittel durch einen entsprechend hohen Druck (der beispielsweise durch die Atmung eines Menschen oder Tieres erzeugt wird) überwunden werden.

Bei Durchführung von Messungen im Durchfluss des Probegases durch die Messkammer können des Weiteren zeitliche Veränderungen in der Zusammensetzung des Probegases in Echtzeit mit hoher Auflösung erfasst werden. Beispielsweise ist es möglich, Änderungen von Isotopenverhältnissen im Atemgas in Echtzeit zu bestimmen, und dies bei Kohlendioxidkonzentrationen des Atemgases im Bereich zwischen 0,08 % und 8 %.

In einer Ausgestaltung der Erfindung ist vorgesehen, dass die Laserfrequenz und deren Variation derart gewählt sind, dass mindestens zwei Absorptionslinien des Probegases in dem definierten Spektralbereich liegen. Dies ermöglicht es beispielsweise, anhand der bei zwei Absorptionslinien erfolgten Lichtabsorption das Verhältnis zweier Isotope des Probegases zu bestimmen. Bei den Isotopen handelt es sich beispielsweise um ¹³CO₂ und ¹²CO₂. Als Isotope werden dabei nicht nur Atome mit gleicher Ordnungszahl, aber unterschiedlicher Massenzahl bezeichnet, sondern auch Moleküle, die solche unterschiedliche Atome enthalten. Statt dem Verhältnis zweier Isotope kann auch das Verhältnis zweier Elemente (mit unterschiedlichen Ordnungszahlen) oder zweier Moleküle anhand von zwei oder mehr Absorptionslinien bestimmt werden.

Die Bestimmung des Verhältnisses zweier Isotope, Elemente oder Moleküle erlaubt darüberhinaus die Bestimmung absoluter Werte der jeweiligen Isotope, Elemente und Moleküle auch bei schwankenden Konzentrationen. Bei beispielsweise der Bestimmung des CO₂ Gehalts in der Atemluft treten beim Atmen Schwankungen in der Konzentration des CO₂ von 0,04% bis 4% auf. Das Schwankungsmaß kann durch die Bestimmung des absoluten Gehalts an ¹²CO₂ (z.B. pro Atemzug) erfasst werden. Damit kann auch der absolute Gehalt des Isotops ¹³CO₂ bestimmt werden, das in einem festen natürlichen Verhältnis zu ¹²CO₂ steht. Zusätzlich können Änderungen aufgrund einer zusätzlichen Metabolisierung von ¹³CO₂ über eine Auswertung der Änderung des Verhältnis der beiden Isotope erfasst werden.

Die hohe Auflösung und Punktdichte der erfindungsgemäßen Messvorrichtung erlauben es, das Verhältnis zweier Isotope, Elemente oder Moleküle in Echtzeit zu bestimmen. Dies ist insbesondere dann von Interesse, wenn sich das Verhältnis mit der Zeit ändert. In einer Ausgestaltung ist der Auswerteinheit für diesen Fall eine Anzeige zugeordnet, die eine Änderung des Verhältnisses über der Zeit darstellt.

Die erfindungsgemäße Messvorrichtung kann spiegellos ausgebildet sein, wobei das vom Laser emittierte Licht die Messkammer genau einmal durchläuft. Hierdurch wird ein einfacher und störungsarmer optischer Aufbau bereitgestellt. Anders als bei der Messkammer der WO 2007/107 366 A1 sind somit keine Spiegel in der Messkammer vorhanden, an denen das Laserlicht mehrfach reflektiert würde. Die Messkammer weist lediglich ein Eintrittsfenster auf, durch das Laserlicht in die Messkammer eintritt, und ein Austrittsfenster, durch das transmittiertes Licht aus der Messkammer austritt.

In einer weiteren Ausgestaltung weist die Messvorrichtung Temperiermittel (insbesondere Heizmittel) auf, die die Messkammer und vorhandene Fenster auf eine konstante Temperatur, die beispielsweise bei über 35 °C liegt, temperieren, insbesondere heizen. Hierdurch wird verhindert, dass eventuell in dem Probegas vorhandener Wasserdampf die Messkammer beschlägt. Ein Kühlen der Messkammer ist ebenso denkbar.

Erfindungsgemäß ist vorgesehen, dass die Messkammer kontinuierlich oder intermittierend von Probegas durchströmt wird. Hierzu weist die Messkammer in einer Ausgestaltung einen offenen Aufbau ohne Ventile oder Luftklappen auf, die den Fluss des Probegases in und aus der Messkammer behindern könnten. Außerdem weist die Messvorrichtung zwischen Gaseintritt in die Messvorrichtung und Gasaustritt aus der Messvorrichtung im Wesentlich einen konstanten Querschnitt für das durchströmende Gas auf Hierdurch wird ein laminarer Fluss an allen Stellen der Messvorrichtung bereitgestellt und verhindert, dass sich an bestimmten Stellen Gas ansammelt und nicht durch neues Probegas verdrängt wird.

In einer Variante weist die Messvorrichtung zumindest abschnittsweise, insbesondere innerhalb der gesamten Messkammer, einen konstanten Querschnitt auf, so dass zumindest abschnittsweise ein laminarer Fluss in der Messvorrichtung gewährleistet wird. Weist beispielsweise die gesamte Messkammer einen konstanten Querschnitt aus, ist innerhalb der gesamten Messkammer im Betrieb im Wesentlichen ein laminarer Fluss des Probegases gewährleistet. Dadurch werden sehr präzise Messungen auf besonders vorteilhafte Weise ermöglicht.

In einer weiteren Ausgestaltung erfolgt eine Zuleitung von Probegas in die Messkammer und ein Abführen von Probegas aus der Messkammer in einer Richtung, die senkrecht ist zur Richtung, in der das Licht die Messkammer durchläuft. Dies stellt sicher, dass die Gaszu- und abfuhr sowie entsprechende Anschlüsse das Laserlicht nicht stören. Gaszu- und Abfuhr sind dabei bevorzugt versetzt angeordnet, so dass das Probegas teilweise in Richtung des Laserstrahls durch die Messkammer strömt.

Die Messvorrichtung ist vorzugsweise derart ausgebildet, dass eine zeitaufgelöste Lichterfassung durch die Detektionsvorrichtung während des Durchströmens des Probegases durch die Messkammer erfolgt. Infrarot-Absorptionsmessungen werden somit in jeder Phase des Gasflusses durchgeführt, insbesondere auch, wenn das Probegas die Messkammer durchströmt. Die Absorptionsmessungen finden im echten Durchfluss (also in Durchflussmesstechnik) statt.

In einer weiteren Ausgestaltung umfasst die Messvorrichtung ein Spirometer, das den Volumenstrom des Probegases, das durch die Messkammer strömt, erfasst. Dabei kann vorgesehen sein, dass das Probegas das Spirometer nach Beströmen der Messkammer durchströmt, für welchen Fall es dann durch das Spirometer aus der Messvorrichtung austritt. Das Spirometer kann aber grundsätzlich an beliebiger Stelle zwischen Gaseintritt in die Messvorrichtung und Gasaustritt aus der Messvorrichtung in dieser angeordnet sein.

Die Messung des Volumenstroms ermöglicht es, absolute Gesamtmengen bestimmter Moleküle einer bestimmten Gasmenge, die beispielsweise der Gasmenge eines Atemzugs eines Menschen oder Tieres entspricht, zu bestimmen. Insbesondere kann aus der Absorption direkt die Konzentration bestimmt werden, da der Extinktionskoeffizient für jede Absorptionslinie bekannt ist und die Länge der Messkammer ebenfalls. Da die Absorption und mittels des Spirometers der Volumenstrom zeitaufgelöst in Echtzeit verfolgt werden können, kann die Gesamtmenge durch Integration des Produktes von Volumen und Konzentration über der Zeit in Echtzeit bestimmt werden.

In einer Ausgestaltung ist des Weiteren eine Vorkammer vorgesehen, durch die das Probegas in die Messkammer strömt. Die Vorkammer ist dabei bevorzugt dazu ausgebildet, das Probegas auf eine bestimmte Temperatur zu erwärmen oder abzukühlen und dadurch den Wasserdampfgehalt des Probegases zu reduzieren.

In einer weiteren Ausgestaltung ist die Vorkammer alternativ oder zusätzlich bevorzugt dazu ausgebildet, den Wasserdampfgehalt des Probegases auf wenigstens 60% relative Luftfeuchtigkeit zu reduzieren. Die Reduktion des Wasserdampfgehaltes erfolgt vorzugsweise durch semipermeable Membranen, die ausschließlich einen Austausch von Wasserdampf (nicht jedoch von anderen Substanzen) erlauben. Die Luft außerhalb der Membran muss eine relative Luftfeuchtigkeit von weniger als 50% relative Luftfeuchtigkeit aufweisen. Ist der Wasserdampfgehalt außerhalb der Vorkammer geringer als innerhalb, so wird der Wasserdampfgehalt des die Vorkammer durchströmenden Probegases reduziert. Die Gesamtfläche der Membran bestimmt, wie viel Gasaustausch stattfinden kann.

Als Beispiel sei die Anwendung der Messvorrichtung für die Atemanalyse genannt, in der ein einzelner Atemzug (insbesondere ein vollständiger Atemzug) untersucht wird. Die Luftfeuchtigkeit in einem Atemzug beträgt oftmals mehr als 90% relative Luftfeuchtigkeit, die durch die semipermeable Membran in der Vorkammer auf weniger als 50% relative Luftfeuchtigkeit reduziert wird. Die aktive Fläche der semipermeablen Membran kann dabei z. B. mehr als 150 cm², insbesondere mehr als 200 cm² und ganz besonders mehr als 250 cm² betragen.

In einer weiteren Ausgestaltung ist die Vorkammer alternativ oder zusätzlich bevorzugt dazu ausgebildet, das Probegas zu homogenisieren. Die Homogenisierung des einzelnen Probegases erfolgt durch verschiedene (mindestens zwei) Abzweigungen unterschiedlicher Länge und Durchmesser, die von Teilen des Probegases durchlaufen werden. Nach dem Bereich der Abzweigungen werden die Teile des Probegases wieder zusammengeführt. Dabei ist es wichtig, dass der Gesamtquerschnitt aller Abzweigungen (also die Summe der Querschnitte der einzelnen Abzweigungen) einen gleich großen Strömungsquerschnitt wie der Rest der Messvorrichtung aufweist, damit durch die Abzweigungen kein erhöhter oder nur geringfügig erhöhter Druckwiderstand für den Fluss des Probegases in der Messvorrichtung erzeugt wird. Die Längen der verschiedenen Abzweigungen, durch die das Probegas fließt, sind so gewählt, dass Probegasvolumina einer bestimmten Volumengröße optimal durchmischt werden. Die Durchmischung findet rein passiv statt und nutzt einzig die Druckdifferenz zum Auslass der Messvorrichtung aus, die das Probegas zum Strömen verleitet.

Als Beispiel sei die Anwendung der Messvorrichtung für die Atemanalyse genannt, in der ein einzelner (insbesondere vollständiger) Atemzug homogenisiert wird. Das Ausatmen erzeugt den Druckunterschied, der das Probegas zum Strömen verleitet. Das durchschnittliche Volumen eines Atemzuges beträgt etwa 500 ml. Schon bei Abzweigungen mit drei verschiedenen Durchmessergrößen mit Verhältnissen d3:d2:d1 = 3:2:1 zeigt der laminare Volumenstrom unterschiedliche Geschwindigkeiten v3 < v2 < v1. Hält man den Gesamtdurchschnitt für die einzelnen Durchmessergrößen d1, d2 und d3 konstant, indem mehrere Abzweigungen mit Durchmessergrößen d1 und d2 gewählt werden, so fließt durch alle Abzweigungen mit gleichem Durchmesser etwa das gleiche Volumen (bei Vernachlässigung der Reibung). Durch die unterschiedlichen Strömungsgeschwindigkeiten des Probegases kann nun durch Auswahl der Abzweigungslänge die gewünschte Volumenmenge (z.B. 500 ml) gut durchmischt werden. Die Anzahl der Abzweigungen beträgt mindestens zwei. Je mehr Abzweigungen verwendet werden, desto homogener kann das Probegas durchmischt werden. Eine gute Durchmischung erlaubt eine präzisere und schnellere Messung von Gasbestandteilen im Probegas. Sie ist z.B. für hochpräzise Messungen mit Durchflussmesstechnik wichtig.

Die Durchmesser der einzelnen Abzweigungen sind vorzugsweise derart ausgewählt, dass eine zweite Abzweigung einen um mindestens 50 %, insbesondere mindestens 60 %, insbesondere mindestens 70 %, insbesondere mindestens 80 %, insbesondere mindestens 90% und ganz besonders mindestens 100% größeren Durchmesser aufweist als eine erste Abzweigung.

Der schmalbandig emittierende Laser weist in einer Ausgestaltung der Erfindung eine Linienbreite von weniger als 0,2 cm⁻¹, insbesondere von weniger als 0,05 cm⁻¹ auf. Je geringer dabei die Linienbreite ist, desto genauer kann ein bestimmter Spektralbereich ausgemessen werden. Der Laser ist in einem Ausführungsbeispiel ein Infrarot-Quantenkaskadenlaser, der beispielsweise Licht im Frequenzbereich zwischen 2200 cm⁻¹ und 2400 cm⁻¹, insbesondere im Bereich zwischen 2295 cm⁻¹ und 2305 cm⁻¹ emittiert.

Zur Variation der Frequenz des Lasers sind Mittel zum Durchstimmen des Lasers vorgesehen, die in einer Ausführungsvariante eine mit einer Modulationsfrequenz periodisch modulierte Spannung am Laserkopf des Lasers anlegen, wobei die angelegte Spannung mit einer kurzfristigen Temperaturerhöhung und damit mit einer Frequenzverschiebung einhergeht. Durch eine entsprechende Spannungsmodulierung lässt sich also eine wiederholte Temperaturerhöhung und Temperaturerniedrigung des Lasers erreichen. Die Durchstimmbarkeit des Lasers liegt dabei bevorzugt zwischen 0,5 cm⁻¹ und 60 cm⁻¹, insbesondere bei 1 cm⁻¹, 2 cm⁻¹, 6 cm⁻¹ oder 20 cm⁻¹. Die Frequenzvariation bestimmt den Spektralbereich, der ausgemessen wird. Die Modulationsfrequenz bestimmt, mit welcher Frequenz ein bestimmter Spektralbereich gemessen wird. Die Modulationsfrequenz liegt in einer Ausgestaltung zwischen 100 und 500 Hz, insbesondere zwischen 10 und 100 Hz, insbesondere bei etwa 50 Hz. Die in dem Laserkopf angelegte Spannung ist in einer Ausgestaltung eine Dreiecksspannung, so dass ein definiertes Frequenzspektrum linear erst nach oben und dann wieder nach unten durchfahren wird. Alternativ kann beispielsweise eine Sägezahnspannung verwendet werden.

Wie bereits ausgeführt, weist die erfindungsgemäße Messvorrichtung eine hohe Zeitauflösung der einzelnen Messungen auf, die mit einer hohen Punktdichte des ausgemessenen Spektrums korreliert. Die Detektionsvorrichtung ist dabei dazu ausgebildet und eingerichtet, pro Spektralbereich, in dem die Laserfrequenz variiert, also während einer Periode der Modukationsfrequenz, mehr als 20 Messpunkte, bevorzugt mehr als 100 Messpunkte, besonders bevorzugt mehr als 500 Messpunkte zu messen.

Das Lasersignal, das der Laser aussendet, ist bevorzugt gepulst und weist in einer Ausgestaltung eine Pulsdauer von weniger als 200 ns, insbesondere von weniger als 100 ns auf. Die Detektionsvorrichtung ist dabei in einer Ausgestaltung dazu ausgebildet und eingerichtet, zu jedem ausgesendeten Lichtpuls des Lasers eine Absorptionsmessung durchzuführen. Jeder Laserpuls führt somit zu einem Absorptions-Messwert.

Weiter kann vorgesehen sein, dass die Detektionsvorrichtung mit einer Frequenz ausgelesen wird, die doppelt so groß ist wie die Frequenz, mit der der Laser Lichtpulse aussendet. Das Auslesen erfolgt also mit der doppelten Laserrepitionsrate. Dies bedeutet, dass nur jeder zweite Auslesevorgang einen gemessenen Lichtpuls betrifft. Die dazwischen liegenden Auslesevorgänge korrelieren mit keinem gemessenen Signal und geben lediglich das Untergrundsignal wieder. Das Untergrundsignal wird bevorzugt direkt abgezogen. Dies ermöglicht es, die Messgenauigkeit weiter zu erhöhen.

Zur Erhöhung der Messgenauigkeit ist in einer Ausführungsvariante des Weiteren vorgesehen, dass das vom Laser emittierte Licht in zwei Teilstrahlen aufgeteilt wird, wobei der eine Teilstrahl durch die Messkammer läuft und der andere Teilstrahl von einer Referenzdetektionsvorrichtung erfasst wird. Die Auswerteinheit wertet die Signale der Referenzdetektionsvorrichtung zur Normierung der Signalstärke des Lasers aus. Hierdurch können Intensitätsschwankungen des Lasers herausgerechnet werden, was die Genauigkeit der durchgeführten Messungen weiter erhöht.

Die erfindungsgemäße Messvorrichtung ist in einer Ausgestaltung dazu eingerichtet, als Probegas das Atemgas eines Menschen oder eines Tieres zu untersuchen. Insbesondere ist die Messvorrichtung geeignet, zeitaufgelöst das Verhältnis der ¹³CO₂ / ¹²CO₂ Isotopenkonzentration im Atemgas des Menschen oder Tieres zu bestimmen. Weiter kann eine quantitative Messung eines Stoffwechselparameters im Atemgas in Echtzeit erfolgen. Beispielsweise ist die Messvorrichtung dazu eingerichtet, die Gesamtmenge von ¹³CO₂ pro Atemzug zu bestimmen. Bei einer Messung von mehreren Atemzügen in Folge kann dies mit einer Genauigkeit von etwa 10 µg erfolgen. Weiter ist die Messvorrichtung dazu eingerichtet, die Kohlendioxidkonzentration des Atemgases im Bereich zwischen 0,08% und 8% im Durchfluss in Echtzeit zu bestimmen.

Eine weitere Anwendung erlaubt es, mittels der erfindungsgemäßen Messvorrichtung die Linienbreite einer Absorptionslinie des Probegases in Abhängigkeit von der Gaskonzentration zu bestimmen. So kann durch die hohe Zeitauflösung, die hohe spektrale Auflösung und die hohe Empfindlichkeit der durchgeführten Absorptionsmessungen die Linienbreite einer betrachteten Absorptionslinie in Abhängigkeit der Gaskonzentration bestimmt werden. Hierzu werden die Linienbreiten bei definierten, vorab eingestellten Gaskonzentrationen gemessen.

Die Erfindung betrifft des Weiteren ein Verfahren zur Untersuchung eines Probegases mittels Infrarot-Absorptionsspektroskopie. Das Verfahren umfasst folgende Schritte:
- Durchstrahlen einer Messkammer mit Licht, das von einem schmalbandigen Laser emittiert wird, dessen Linienbreite kleiner oder gleich der Breite einer zu messenden Infrarot-Absorptionslinie eines in der Messkammer befindlichen Probegases ist, wobei die Laserfrequenz periodisch innerhalb eines definierten Spektralbereichs variiert wird und die Laserfrequenz und deren Variation derart gewählt werden, dass mindestens eine zu messende Infrarot-Absorptionslinie des Probegases in dem definierten Spektralbereich liegt,
- zeitaufgelöstes Erfassen des vom Laser emittierten und die Messkammer durchstrahlten Lichts, wobei eine einzelne Absorptionsmessung innerhalb von 10⁻⁵ s oder schneller durchgeführt wird, und
- Auswerten der erfassten Signale hinsichtlich einer in der Messkammer erfolgten Lichtabsorption, wobei die Lichtabsorption innerhalb des definierten Spektralbereichs frequenzaufgelöst bestimmt wird.

Die Erfindung wird nachfolgend unter Bezugnahme auf die Figuren der Zeichnungen anhand mehrerer Ausführungsbeispiele näher erläutert. Es zeigen:
- Fig. 1: ein Ausführungsbeispiel einer Messvorrichtung zur Untersuchung eines Probegases mittels Infrarot-Absorptionsspektroskopie;
- Fig. 2: die Messung einer ¹³CO₂ Absorptionslinie bei 2297,19 cm⁻¹ mittels der Messvorrichtung der Figur 1, wobei die Absorption in Abhängigkeit von der Frequenz in Wellenzahlen innerhalb eines ausgemessenen Spektralbereichs dargestellt ist;
- Fig. 3: die gleichzeitige Messung der ¹²CO₂ und der ¹³CO₂ Absorptionslinien im Verlauf von zwei aufeinander folgenden Atemzügen, wobei die Absorption zum einen gegen die Zeit und zum anderen gegen die Frequenz in Wellenzahlen dargestellt ist;
- Fig. 4: das Verhältnis der ¹³CO₂ zur ¹²CO₂ Konzentration im Messbereich zwischen 0 DOB und 300 DOB, wobei die Abzisse ein eingestelltes Konzentrationsverhältnis von Testgasen und die Ordinate durch die Messvorrichtung der Figur 1 gemessene Werte darstellt;
- Fig. 5: die ¹²CO₂ Zunahme eines Probanden nach Einnahme von 13C-markiertem Methacetin in Abhängigkeit von der Zeit;
- Fig. 6: Linienbreiten der CO₂ Absorptionslinien in Abhängigkeit von der CO₂ Konzentration des Probegases, bei gleichbleibendem Druck; und
- Fig. 7: eine schematische Darstellung eines Messablaufs zur Leberleistungsbestimmung unter Verwendung der Messvorrichtung der Figur 1.

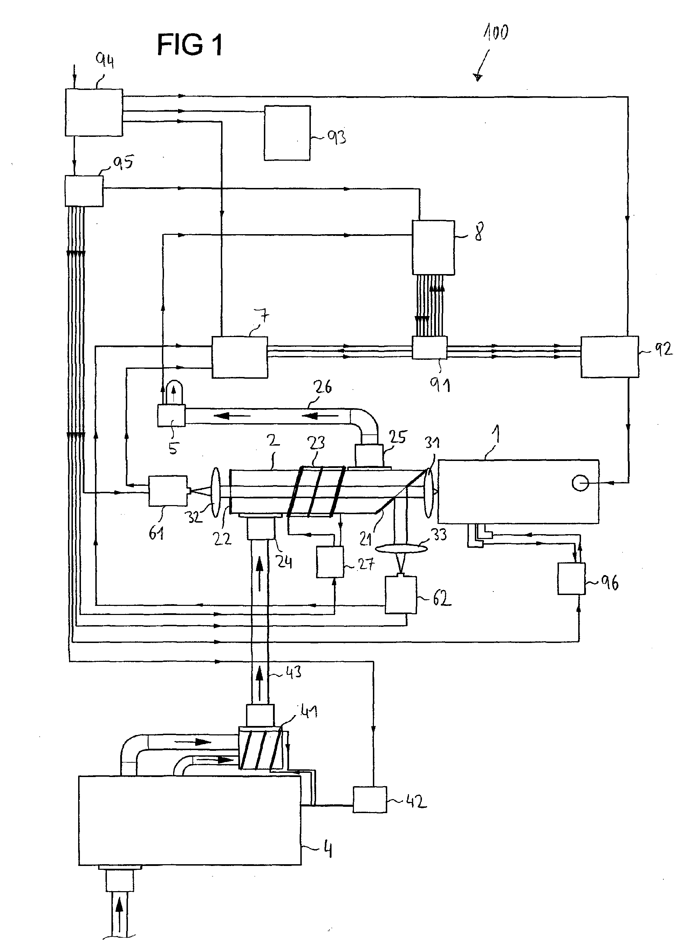

Die Figur 1 zeigt eine Messvorrichtung 100 zur Untersuchung eines Probegases mittels Infrarot-Absorptionsspektroskopie. Die Vorrichtung 100 umfasst einen Laser 1, eine Messkammer 2, zwei Detektoren 61, 62, eine Vorkammer 4, ein Spirometer 5, einen Ladungsverstärker 7 und eine Auswerteinheit 8.

Der Laser 1 ist ein Infrarot-Quantenkaskadenlaser (QCL), der eine Linienbreite unter 0,2 cm⁻¹, insbesondere eine Linienbreite von 0,05 cm⁻¹, aufweist. Die Grundfrequenz des Quantenkaskadenlasers wird über dessen Temperatur eingestellt. Diese wird auf einer Zeitskala von etwa 0,05 bis 0,5 s mittels einer Laser-Steuereinheit 92 geregelt. Die Laserfrequenz ist zusätzlich periodisch innerhalb eines definierten Spektralbereichs variierbar. Hierzu wird eine Spannung, die im Folgenden auch als "Sweep-Spannung" bezeichnet wird, mittels der Laser-Steuereinheit 92 zusätzlich am Quantenkaskadenlaser 1 angelegt. Die Sweep-Spannung und ein damit korrespondierender Sweep-Strom sorgen für eine kurzfristige Temperaturerhöhung während des zusätzlichen Stromflusses im Laser und verschiebt damit die Frequenz. Die Parameter des Lasers sind derart eingestellt, dass vorzugsweise direkt nach Beendigung des Stromflusses wieder die Grundfrequenz ausgegeben wird.

Die Sweep-Spannung wird kontinuierlich, beispielsweise mittels einer Dreiecksspannung oder Sägezahnspannung erhöht und dann wieder abgesenkt, wodurch eine kontinuierliche Frequenzvariation stattfindet. Aufsetzend auf der Grundfrequenz wird die Frequenz des Lasers 1 somit periodisch variiert. Die Frequenzvariation korreliert mit einer Durchstimmbarkeit des Lasers, die bei mindestens 0,5 cm⁻¹ liegt. Beispiele für die Breite der Durchstimmbarkeit sind 1, 2, 6, 20 oder 60 cm⁻¹. Die Durchstimmbarkeit gibt dabei einen Spektralbereich an, innerhalb dessen die Laserfrequenz variiert wird. Die Modulationsfrequenz, mit der die Laserfrequenz periodisch variiert wird, liegt im Bereich zwischen 1 und 500 Hz. Sie definiert, wie häufig der betrachtete Spektralbereich ausgemessen wird. Im Folgenden wird beispielhaft von einer Modulationsfrequenz von 50 Hz ausgegangen.

Der Laser 1 ist ein gepulster Laser, der Lichtsignale mit einer Pulsdauer von weniger als 200 ns, insbesondere von 100 ns oder sogar kürzer aussendet. Damit ist die maximale Zeitauflösung einer Messung auf 200 ns bzw. 100 ns begrenzt. Die Verwendung relativ kurzer Pulsdauern sorgt insofern für spektral schmale Linienbreiten, als bei lang anhaltenden Pulsen eine Linienverbreiterung auf Grund einer Temperaturerhöhung erfolgt, die mit einem vergleichsweise langen Aussenden von Laserlicht verbunden ist.

Die Laserrate, das heißt die Anzahl der Pulse, die pro Sekunde ausgesandt wird, liegt beispielsweise zwischen 10 und 100 kHz. Im Folgenden wird beispielhaft eine Laserrate von 50 kHz angenommen.

Der Laser 1 ist in einem geschlossenen Gehäuse angeordnet, das einen Kontakt mit der Außenluft verhindert. Hierzu ist er beispielsweise in einem TO3-Gehäuse angeordnet. Eine Wasserkühlung 96 sorgt für eine Kühlung des Lasers 1.

Über die Laser-Steuereinheit 92 wird des Weiteren das Treibersignal für den Quantenkaskadenlaser 1 angelegt.

Die Messkammer 2 weist ein abgeschrägtes Eintrittsfenster 21, durch das das Laserlicht in die Messkammer 2 eintritt, und ein senkrecht zum Strahlengang angeordnetes Austrittsfenster 22 auf. Das vom Laser 1 ausgestrahlte Licht wird über eine antireflexbeschichtete Linse 31 auf das abgeschrägte Eintrittsfenster 21 gelenkt. Am Eintrittsfenster 21 wird das Licht in zwei Teilstrahlen aufgeteilt. Der transmittierte Strahl durchquert die Messkammer 2, verlässt die Messkammer 2 durch das Austrittsfenster 22 und fällt nach Fokussierung durch eine weitere antireflexbeschichtete Linse 32 auf einen ersten Detektor 61. Der reflektierte Strahl fällt über eine weitere antireflexbeschichtete Linse 33 auf einen zweiten Detektor 61. Die antireflexbeschichteten Linsen 31, 32, 33, die beispielsweise aus ZnSe, Saphir, CaF₂ oder BaF₂ bestehen, sind direkt mit dem Laser 1 bzw. den jeweiligen Detektoren 61, 62 verbunden, so dass der Messaufbau nur aus vier Komponenten besteht, nämlich dem Laser, den beiden Detektoren und der Messkammer. Dies führt zu einem einfachen, robusten Aufbau.

Das Laserlicht durchquert die Messkammer 1, die spiegellos ausgebildet ist, nur einmal. Dies erhöht weiter die Einfachheit und damit Störungsunanfälligkeit des Messaufbaus.

Die Messkammer 2 umfasst eine Temperiereinrichtung 23, die insbesondere als Heizeinrichtung ausgestaltet ist und die über einen Temperaturregler 27 geregelt wird.

Die Temperiereinrichtung 23 sorgt für eine konstante Temperatur innerhalb der Messkammer, die bei beispielsweise 35 °C oder darüber liegt. Dies verhindert, dass eventuell in dem Probegas, das die Messkammer 2 durchströmt, vorhandener Wasserdampf die Messkammer 2 beschlägt. Die konstante Temperatur kann auch unterhalb der Umgebungstemperatur liegen.

Zur Zuführung eines Probegases in die Messkammer 2 weist diese einen ersten Anschluss 24 auf. Der Anschluss 24 ist am Messkammergehäuse angeordnet, das beispielsweise aus Aluminium besteht. Über den Anschluss 24 wird der Messkammer 2 das Probegas von einer Vorkammer 4 über einen Schlauch 43 oder dergleichen zugeführt. Der Vorkammer 4 ist ebenfalls eine Heizeinrichtung 41 zugeordnet, die über einen Temperaturregler 42 eine Regelung der Temperatur des der Messkammer 2 zugeführten Probegases vornimmt. Hierdurch wird bereits in der Vorkammer 4 das Probegas erwärmt und in seinem Wasserdampfgehalt reduziert. Statt der Heizeinrichtung 41 könnte auch eine Temperiervorrichtung 41 verwendet werden, die das Probegas in der Vorkammer 4 bei Bedarf auch kühlen könnte.

Weiter weist die Messkammer 2 einen Anschluss 25 für das aus der Messkammer 2 ausströmende Probegas auf. Das Probegas strömt dabei beispielsweise durch einen Schlauch 26 oder dergleichen zu einem Spirometer 5, das den Volumenstrom, der durch die Messkammer 2 fließt, bestimmt. Nach Durchströmen des Spirometers 5 tritt das Probegas aus der Messvorrichtung in die Umgebung aus, wobei das Spirometer 5 auch an anderer Stelle in der Messvorrichtung angeordnet sein kann.

Das Probegas strömt in die Messkammer 2 senkrecht zu der Richtung, in der das Laserlicht die Messkammer 2 durchstrahlt. Ebenso strömt es senkrecht zur letztgenannten Richtung aus der Messkammer 2 wieder heraus. Dabei sind die Anschlüsse 24, 25 versetzt am Messkammergehäuse angeordnet.

Der gesamte Messaufbau der Messvorrichtung ist ein offener Aufbau ohne Ventile oder Luftklappen, die den Fluss des Probegases behindern könnten. Vielmehr kann das Probegas den beschriebenen Aufbau ungehindert durchströmen. Dabei ist vorgesehen, dass der Querschnitt in der Zuleitung 43, der Messkammer 2 sowie der Ableitung 26 im Wesentlichen konstant ist, so dass an allen Stellen ein laminarer Fluss gewährleistet ist und keine Gasansammlungen an bestimmten Stellen erfolgen. Vielmehr verdrängt Probegas, das über die Vorkammer 4 in die Messkammer 2 eintritt, komplett das zuvor vorhandene Probegas aus dem Messaufbau. Das Probegas strömt durch die Vorkammer 4 in die Messkammer 2 und von der Messkammer 2 durch das Spirometer 5 wieder aus der Messvorrichtung hinaus.

Die Messungen werden bei Normaldruck durchgeführt. Die Messkammer 2, die Vorkammer 4, die Zuleitung 43, die Ableitung 26 und das Spirometer 5 derart ausgeführt, dass sie bis zu einem Überdruck von bis zu 200 mbar im Vergleich zum Normaldruck dicht sind. Liegt kein Druckunterschied zwischen dem Gaseinlass 24 und dem Gasauslass 25 vor, kann das Probegas bis zu mehreren 10 Minuten unverändert in der Messkammer 2 verbleiben.

Die nachfolgend noch näher beschriebenen Infrarot-Absorptionsmessungen werden in jeder Phase des Gasflusses durch die Messkammer 2 durchgeführt, insbesondere auch, wenn das Probegas die Messkammer 2 durchströmt. Die durchgeführten Messungen erfolgen in echtem Durchfluss. Aufgrund des offenen Aufbaus der Messkammer 2 kann das Probegas in der Messkammer 2 dabei beliebig schnell ausgetauscht werden.

Wie noch ausgeführt wird, ist die beschriebene Messvorrichtung dazu geeignet und einrichtbar, als Probegas das Atemgas eines Menschen oder Tieres zu messen. Im Falle der Verwendung von Atemgas als Probegas sorgt der Atemdruck dafür, dass der neue Atemzug den alten Atemzug aus der Messkammer verdrängt und dabei der neue Atemzug in Echtzeit gemessen wird. Somit wird die Atemgasprobe allein durch die Atmung für jeden Patienten individuell so schnell wie nötig in der Messkammer ausgetauscht, wobei Messungen in Echtzeit im Durchfluss erfolgen. Der Atemwiderstand des Messgerätes ist dabei derart ausgelegt, dass er für den Gasfluss bei weniger als 60 mbar liegt.

Bei den Detektoren 61, 62 handelt es sich um MCT-Detektoren. Solche Detektoren sind Halbleiterdetektoren auf der Basis von Quecksilber(II)Cadmium(II)Tellurit. Die Detektoren 61, 62 sind bevorzugt peltiergekühlt, wodurch ein Verzicht auf mit flüssigem Stickstoff gekühlte Detektoren möglich ist, bei trotzdem hoher Sensitivität. Der Verzicht auf flüssigem Stickstoff zur Kühlung erweitert den Einsatzbereich der Messvorrichtung, beispielsweise im klinischen Alltag.

Beide Detektoren 61, 62 werden quasi zeitgleich ausgelesen. Jeder Detektor 61, 62 misst dabei das gesamte Spektrum des durch den Laser 1 ausgesandten Lichts. Hierdurch werden Fehler durch Variation der Detektorsensibilität von Detektor zu Detektor vermieden.

Das von den Detektoren 61, 62 ausgelesene Signal wird zunächst in einem Leitungsverstärker 7 für jeden Detektor 61, 62 getrennt verstärkt und integriert. Das verstärkte Signal wird dann jeweils über einen Adapter 91 einer Auswerteinheit 8 zugeführt, die beispielsweise mittels eines üblichen PCs und geeigneter Software realisiert ist. Das Signal des Detektors 62 dient dabei allein der Normierung der Signalstärke. So können Intensitätsschwankungen am Detektor 61, die durch Intensitätsschwankungen des Lasers 1 verursacht sind, mittels des Signals des Detektors 62 korrigiert werden. Dies erhöht die Genauigkeit der Auswertung.

Die Auswerteinheit 8 empfängt des Weiteren Signale vom Spirometer 5. Auch kann vorgesehen sein, dass der Auswerteinheit 8 über einen nicht dargestellten Sensor oder die Temperaturregler 42, 27 die Temperatur des Probegases und/oder die Temperatur in der Messkammer 2 mitgeteilt wird. Der Auswerteinheit 8 ist ein Monitor 95 zugeordnet.

Die Auswerteinheit 8 erzeugt Steuersignale an die Laser-Steuereinheit 92 in Bezug auf das Sweep-Signal, die Lasertemperatur und die Triggerfrequenz. Der Monitor 95 kann dazu dienen, eine Auswertung der vorgenommenen Absorptionsmessungen graphisch darzustellen. Die Auswerteinheit 8 kann des Weiteren an ein Telekommunikationsnetz, z.B. das Internet und/oder ein Telefonnetz angeschlossen sein.

Ein Netzteil 95, das über einen Transformator 94 an einen Netzstecker angeschlossen ist, sorgt für eine elektrische Versorgung der verschiedenen Elemente der Messvorrichtung.

Wie ausgeführt, wird die Frequenz des Lasers 1 periodisch variiert. Der dabei durchfahrene Spektralbereich wird durch die Sweep-Spannung festgelegt. Bei einer Laserrate von 50 kHz und einer Sweep-Spannung von 50 Hz können pro durchfahrenen Spektralbereich 1.000 Laserpulse gemessen werden. Der betrachtete Spektralbereich wird dabei 50 mal pro Sekunde ausgemessen. Die Messungen können über eine bestimmte Zeit, beispielsweise über die Länge eines Atemzuges gemittelt werden.

Der Detektor 61 ist derart ausgebildet, dass er eine einzelne Absorptionsmessung innerhalb von 10⁻⁵ Sekunden oder schneller durchführt, insbesondere innerhalb von 10⁻⁶ Sekunden oder schneller. Ein gesamter Frequenzbereich, d. h. der Frequenzbereich des durch die Sweep-Spannung definierten Spektralbereichs, kann in etwa 0,002 bis 1 Sekunde abgetastet werden. Im genannten Ausführungsbeispiel liegt die Punktdichte pro Spektralbereich (pro Sweep) bei 1.000 Punkten. Jeder Laserpuls wird detektiert und in einen Messpunkt umgewandelt. Alternativ kann die Punktdichte auch geringer gewählt werden, bei etwa 500 Punkten oder auch nur 20 Punkten pro Spektralbereich.

Die hohe Punktdichte beim Durchfahren des durchmessenden Spektralbereichs verbunden mit der kleinen spektralen Linienbreite des Lasers 1 ermöglicht eine spektrale Auflösung von kleiner als 0,02 cm⁻¹. Dies bedeutet, dass die Absorptionsbanden der vermessenen Gasprobe sehr genau aufgenommen und analysiert werden können. Durch Entfaltung oder andere mathematische verfahren kann die spektrale Auflösung ggf. noch weiter verbessert werden.

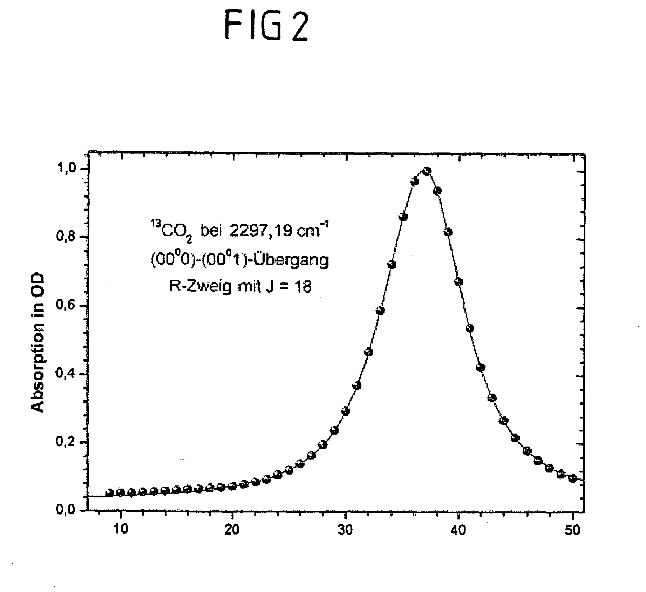

Die Figur 2 zeigt die Messung einer ¹³CO₂-Absorptionslinie, die bei einer Frequenz (in Wellenzahlen) von 2.297,19 cm⁻¹ liegt. Die Abzisse gibt die Änderung der Frequenz (aufgrund der Sweep-Spannung) gegenüber der Grundfrequenz des Lasers 1 an. Die Absorption ist in OD (optische Dichte) angegeben. Die Punktdichte ist hoch genug, um die Absorptionslinie sehr genau bestimmen zu können. Es kann vorgesehen sein, die Kurve zu fitten. Eine Anpassung der Absorptionslinie kann beispielsweise mit Lorenzkurven erfolgen.

Die Datenaufnahme erfolgt jeweils mittels einer Analog/Digital-Karte, die in der Auswerteinheit 8 angeordnet ist und pro Mikrosekunde einen Datenpunkt oder mehr aufnimmt. Die Auflösung ist dabei besser als 12 bit.

Bei einer Laserrate von 50 kHz, einer Modulationsfrequenz des Sweep-Stroms von 50 Hz werden 50 Spektren pro Sekunde gemessen. Pro Spektrum werden 1.000 Punkte gemessen.

Die Ausleserate der Detektoren 61, 62 ist bevorzugt derart gewählt, dass sie doppelt so groß ist wie die Laserrate. Bei einer Laserrate von 50 kHz beträgt die Ausleserate der Detektoren 61, 62 somit 100 kHz. Dies führt dazu, dass nur bei jedem zweiten Auslösevorgang ein detektiertes Lichtsignal ausgelesen wird. Die dazwischen stattfindenden Messungen betreffen den Hintergrund bzw. Rauschen. Das Auslesen der Detektoren 61, 62 mit der doppelten Laserrate ermöglicht es, den Hintergrund bei jeder Lichtmessung sofort abzuziehen. Dies erfolgt bevorzugt in der Auswerteinheit 8 und erhöht weiter die Genauigkeit der Messung.

Es werden mit der beschriebenen Messvorrichtung Absorptionsspektren für einen definierten Spektralbereich in Echtzeit gemessen, da pro Sekunde eine Mehrzahl von Spektren aufgenommen werden kann. Wahlweise kann dabei über eine Mehrzahl von Spektren gemittelt werden, was die Genauigkeit der Messung weiter erhöht. Es wird damit in Echtzeit eine Detektion von Veränderungen der Zusammensetzung eines Probegases möglich.

Um eine hohe Genauigkeit zu erhalten, ist eine hohe Frequenzstabilität erforderlich. Dies wird dadurch erreicht, dass die Temperatursteuerung der Laser-Steuereinheit 92 von einer Messsoftware nachgesteuert wird. Dabei wird die Temperatur in kleinen Schritten nachgeregelt, so dass immer dasselbe Gasabsorptionsmaximum (zum Beispiel von ¹²CO₂) an der gleichen Position im Messbereich liegt. Des Weiteren ist vorgesehen, dass auch die Sweep-Spannung nachgeregelt wird, so dass die weiteren Absorptionslinien an der gewünschten Position im Messbereich liegen. Dadurch werden die Messungen optimal reproduzierbar und können gemittelt werden. Durch große Mittelungszahlen wird das Signal-Rausch-Verhältnis verbessert. Auch erlaubt die Messsoftware bevorzugt eine automatische Erkennung, ob die Laserleistung nachlässt und wann der Laser 1 ausfällt. Die entsprechende Messsoftware kann Teil der Auswerteinheit 8 oder der Laser-Steuereinheit 92 sein.

Weiter kann vorgesehen sein, dass eine schnelle Auswertung der Spektren über eine Integration von speziellen Frequenzbereichen erfolgt, die einzelnen Absorptionslinien zuzuordnen sind. Die Aufnahme von Eichgraden mit Gasproben, die in ihrer Zusammensetzung bekannt sind, erlaubt dabei die einfache und genaue Bestimmung des Offsets ohne ein Fitten der Daten. Da die Frequenzstabilität reproduzierbar eingestellt ist, können gezielt Frequenzbereiche adressiert werden, aus denen die Konzentrationen hochgenau bestimmt werden. Ein Fitten der großen Datenmengen würde möglicherweise den Messprozess verlangsamen und ist daher nicht zwingend erforderlich.

Durch die hohe Selektivität der Messung kann die Messung unabhängig von Testgasen wie Sauerstoff oder anderen Narkosegasen erfolgen. Mit der vorhandenen hohen spektralen Auflösung können Einflüsse jedes anderen Gases abgetrennt werden.

Die Messvorrichtung ist im dargestellten Ausführungsbeispiel dazu optimiert, Leberleistungsmessungen nach Verabreichung von ¹³C-markiertem Methacetin durchzuführen. Das zusätzlich metabolisierte ¹³CO₂ wird in der Atemluft nachgewiesen.

Dies ist ausführlich in der WO 2007/000145 A2 beschrieben, auf die insofern Bezug genommen wird.

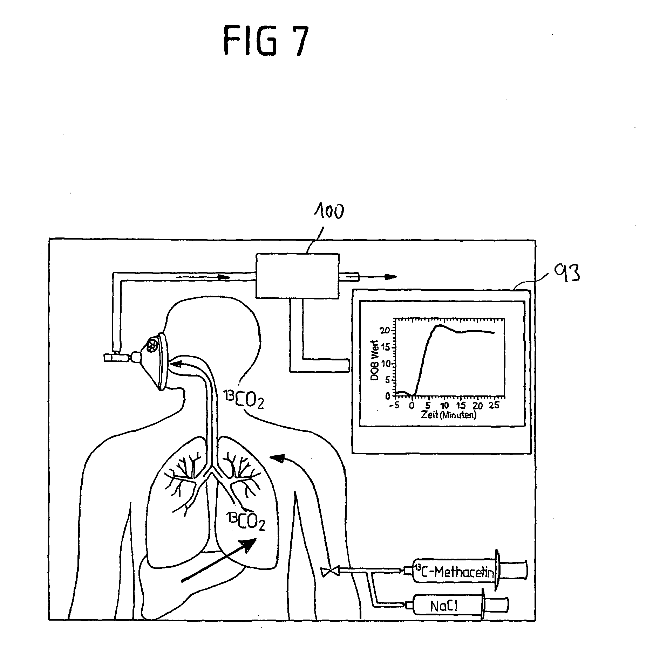

Wie in der Figur 7 schematisch dargestellt ist, trägt ein zu untersuchender Patient eine Atemmaske, die ein Lufteinlassventil und ein Luftauslassventil aufweist. Dadurch wird die Einatemluft von der Ausatemluft getrennt. Ausgeatmete Luft kann nicht wieder eingeatmet werden. Am Luftauslassventil wird ein Plastikschlauch angeschlossen, der die gesamte Atemluft an die Messvorrichtung 100 leitet und der mit der Vorkammer 4 verbunden ist. Die Maske und die Schläuche sind ebenfalls bis zu einem Überdruck von bis zu 200 mbar dicht, so dass die gesamte Atemluft durch die Messvorrichtung 100 strömt.

Dabei erkennt eine Messsoftware automatisch, falls die Maske nicht richtig auf dem Patientengesicht sitzt bzw. verrutscht ist. Auch erkennt die Messsoftware, ob der Patient noch atmet oder nicht und gibt gegebenenfalls eine Warnung aus. Des Weiteren ist vorgesehen, dass die Messsoftware erkennt, wann die Messung beendet werden kann. Eine solche Messsoftware kann ebenfalls in die Auswerteinheit 8 integriert sein.

Die Laserfrequenz des Lasers 1 und der durch die Sweep-Spannung definierte Spektralbereich sind nun so gewählt, dass mindestens zwei Absorptionslinien des Probegases in dem definierten Spektralbereich liegen. Im betrachteten Beispiel sind dies eine Absorptionslinie von ¹³CO₂ und eine Absorptionslinie von ¹²CO₂. Nach Verabreichung von ¹³C-markiertem Methacetin wird dieses in der Leber abgebaut und ist in der Atemluft nachweisbar. Der Abbau korreliert mit einem Anstieg von ¹³CO₂ in der Atemluft, was zu einem veränderten Verhältnis von ¹³CO₂ zu ¹²CO₂ führt. Das Verhältnis wird durch die Messvorrichtung 100 anhand der Absorptionmessungen bestimmt und dessen zeitlicher Verlauf auf einem Monitor 93 dargestellt.

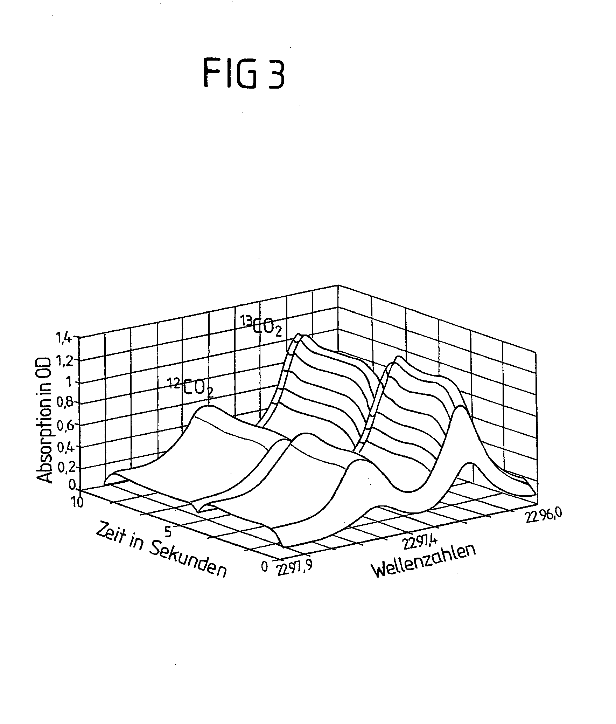

Die Figur 3 zeigt die gleichzeitige Messung der ¹²CO₂ und ¹³CO₂ Absorptionslinien im Verlauf von zwei aufeinander folgenden Atemzügen. Die Absorptionsänderung ist in OD (Optische Dichte) angezeigt. Sie ist sowohl gegen die Zeit in Sekunden als auch gegen die Frequenz in Wellenzahlen aufgenommen und dargestellt. Man sieht deutlich die starken Variationen der Absorption und damit der Konzentration.

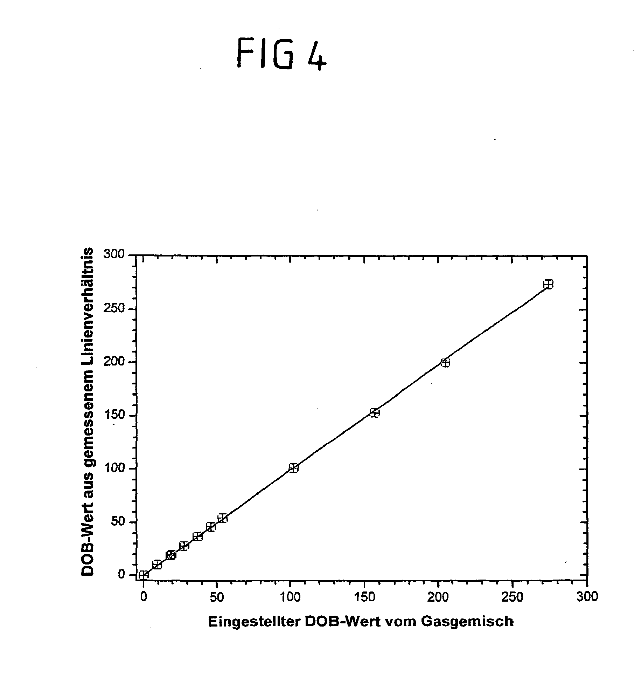

Die Figur 4 verdeutlicht die Genauigkeit, mit der das Verhältnis der ¹³CO₂/¹²CO₂ Konzentration gemessen werden kann. Die Konzentrationsverhältnisse wurden mit sehr genau charakterisierten Testgasen eingestellt und diese Verhältnisse mittels Testmessungen verifiziert. Die Abszisse zeigt dabei einen durch Testgase eingestellten DOB-Wert. Die Ordinate zeigt den gemessenen DOB-Wert, der aus den Linienverhältnissen gemäß Figur 3 bestimmt wird. Dabei bezeichnet 1 DOB eine Änderung des ¹³CO₂ zum ¹²CO₂ Verhältnisses um ein Tausendstel über dem natürlichen Verhältnis. Es zeigt sich, dass das Verhältnis der ¹³CO₂ zur ¹²CO₂ Konzentration mit einer Genauigkeit von besser als 5 DOB pro Atemzug - bei Messung von mehreren Atemzügen in Folge - bestimmt werden kann.

Der Messbereich erstreckt sich von 0 DOB bis über 1000 DOB im Konzentrationsbereich von 0,08% bis 8% CO₂. Über den gesamten Bereich wird das Verhältnis mit höchster Genauigkeit gemessen.

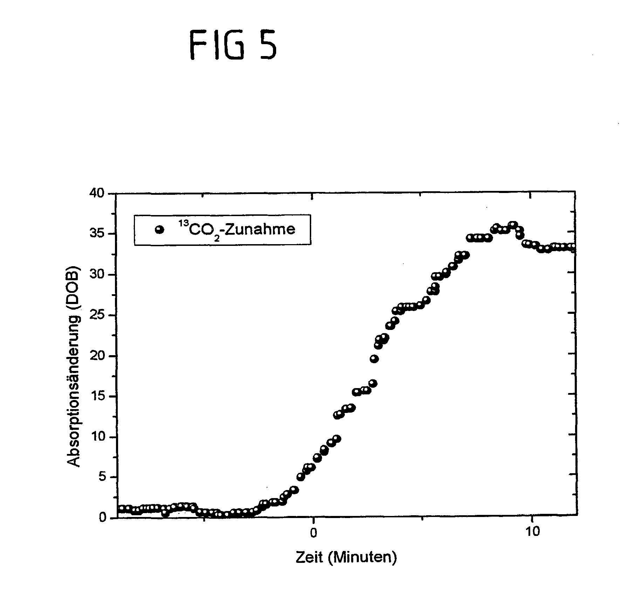

Die Figur 5 zeigt die Leberleistungsbestimmung nach Einnahme von ¹³C-markiertem Methacetin. Dargestellt ist die ¹³CO₂-Zunahme eines Probanden nach Einnahme, die mit einer erhöhten Absorptionsänderung der ¹³CO₂ Absorptionslinie einhergeht. Jeder Atemzug wurde vermessen und entspricht einem Messpunkt (d.h. die bei einem Atemzug durchgemessenen Spektren und das aus diesen ermittelte Verhältnis wurden zu einem Messpunkt gemittelt). Der Anstieg und das Maximum der Absorptionsänderung sind klar und quantitativ genau bestimmbar. Das Diagnostikum wurde etwa bei -3 Minuten verabreicht.

In entsprechender Weise kann auch das Verhältnis anderer Isotope, Elemente oder Moleküle bestimmt werden.

Die erfindungsgemäße Messvorrichtung erlaubt neben der Messung des Verhältnisses bestimmter Isotope auch andere Auswertungen. Beispielsweise kann die Gesamtmenge eines Stoffwechselprodukts, beispielsweise von ¹³CO₂ im Atemzug gemessen werden. So wird der Volumenstrom, der durch die Messkammer 2 fließt, mit dem Spirometer 5 bestimmt. Da das Volumen der Messkammer 2 konstant ist und die Absorption zeitaufgelöst bestimmt wird, kann die Kohlendioxidmenge, die durch die Messkammer fließt, durch Integration über der Zeit bestimmt werden. Insbesondere kann aus der Absorption direkt die Konzentration bestimmt werden kann, da der Extinktionskoeffizient für jede Absorptionslinie bekannt ist, ebenso wie die Länge der Messkammer. Da die Messvorrichtung es ermöglicht, die Absorption zeitaufgelöst in Echtzeit und ebenfalls den Volumenstrom zeitaufgelöst in Echtzeit zu verfolgen, kann die Gesamtmenge über eine Integration des Produktes von Volumen und Konzentration über der Zeit in Echtzeit bestimmt werden.

Durch die Messung der ¹³CO₂ Konzentration und der ¹²CO₂ Konzentration kann somit die Kohlendioxidmenge, die in der Messkammer ist, getrennt für ¹³CO₂ und ¹²CO₂ bestimmt werden. Insbesondere kann die Gesamtmenge von ¹³CO₂- bei Messung von mehreren Atemzügen in Folge - auf etwa 10 µg genau pro Atemzug bestimmt werden.

Eine weitere Anwendung bestimmt die Erfassung der Veränderung der CO₂ Absorptionslinien bei variierender Konzentration des CO₂ in der Atemluft, bei gleichbleibendem Druck in der Atemluft. Mit zunehmender Gaskonzentration und/oder sich veränderndem Partialdruck ändert sich die Linienbreite des Absorptionslinien mittels an sich bekannter Linienverbreiterungsmechanismen. Die Linienbreite kann bei unterschiedlichen bekannten CO₂ Konzentrationen mittels der Messvorrichtung gemessen werden, vergleiche die Absorptionslinien der Figuren 2 und 3.

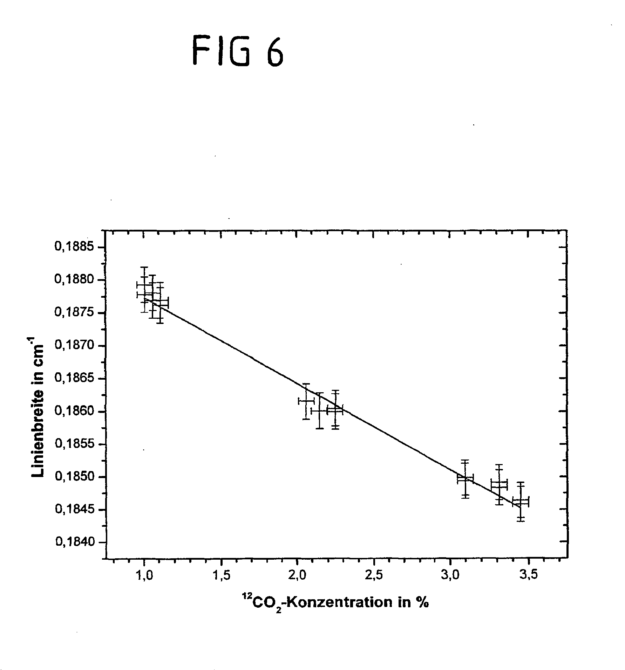

Die Figur 6 zeigt die gemessene Linienbreite in Abhängigkeit von der ¹²CO₂ Konzentration des Atemgases in Prozent. Die ermittelte Abhängigkeit kann zur weiteren Fehlerreduktion ausgewertet werden.

Der Frequenzbereich für die Messung von ¹³CO₂ und ¹²CO₂ liegt zwischen 2200 und 2400, insbesondere bei 2295 bis 2305 cm⁻¹. Allgemein wird bevorzugt ein Laser 1 verwendet, der im Frequenzbereich zwischen 2 µm bis 12 µm Licht emittiert.

Der Einsatz der beschriebenen Messvorrichtung ist nicht auf die Messung des CO₂ Gehaltes der Atemluft beschränkt. Mit dem beschriebenen Messgerät kann jede beliebige Gasprobe untersucht werden. Dabei kann z.B. ein Isotopenverhältnis beliebiger Gase hochempfindlich und sehr genau in Echtzeit bestimmt werden. Die erfindungsgemäße Messvorrichtung ermöglicht eine quantitative, dynamische und zeitaufgelöste Messung von Stoffwechselparametern in Echtzeit. Dabei können auch Belastungsanalysen eines Menschen oder Tieres in Echtzeit durchgeführt werden.

## Patentansprüche

1. Messvorrichtung (100) zur Untersuchung eines Probegases mittels Infrarot-Absorptionsspektroskopie, die aufweist:
- eine Messkammer (2) mit dem zu untersuchenden Probegas,
- einen Laser (1), der derart in Bezug auf die Messkammer (2) angeordnet ist, dass vom Laser emittiertes Licht die Messkammer (2) durchstrahlt,
- eine Detektionsvorrichtung (61), die das vom Laser (1) emittierte und die Messkammer (2) durchstrahlte Licht erfasst, und
- eine Auswerteinheit (8), die von der Detektionsvorrichtung (61) erzeugte Signale hinsichtlich einer in der Messkammer (2) erfolgten Lichtabsorption auswertet, wobei
- der Laser (1) ein schmalbandig emittierender Laser ist, dessen Linienbreite kleiner oder gleich der Breite einer zu messenden Infrarot-Absorptionslinie des Probegases ist und bei weniger als 0,2 cm⁻¹ liegt, wobei
- der Laser (1) derart ausgebildet und eingerichtet ist, dass die Laserfrequenz periodisch innerhalb eines definierten Spektralbereichs variiert wird, wobei die Laserfrequenz und deren Variation derart gewählt sind, dass mindestens eine zu messende Infrarot-Absorptionslinie des Probegases in dem definierten Spektralbereich liegt, und wobei
- die Detektionsvorrichtung (61) derart ausgebildet und eingerichtet ist, dass sie das vom Laser (1) emittierte und die Messkammer (2) durchstrahlte Licht derart zeitaufgelöst erfasst, dass die Lichtabsorption innerhalb des definierten Spektralbereichs frequenzaufgelöst bestimmbar ist,
**dadurch gekennzeichnet,**
**dass** die Detektionsvorrichtung (61) dazu ausgebildet und eingerichtet ist, in dem definierten Spektralbereich eine Mehrzahl von Messpunkten zu erfassen, die jeweils einer einzelnen Absorptionsmessung entsprechen, und jede einzelne Absorptionsmessung innerhalb von 10⁻⁵ s oder schneller durchzuführen, und dass die Messvorrichtung (100) dazu geeignet und einrichtbar ist, als Probegas, das Atemgas eines Menschen oder Tieres zu messen, wobei die Messvorrichtung (100) einen offenen Aufbau ohne Ventile, die den Fluss des Probegases behindern könnten, und einen konstanten Querschnitt für das durchströmende Gas zwischen Gaseintritt in die Messvorrichtung und Gasaustritt aus der Messvorrichtung aufweist, so dass das Atemgas allein durch die Atmung des Menschen oder des Tieres in der Messkammer (2) austauscht und der Atemwiderstand der Messvorrichtung (100) bei weniger als 60 mbar liegt.

2. Messvorrichtung (100) nach Anspruch 1, **dadurch gekennzeichnet, dass** die Laserfrequenz und deren Variation derart gewählt sind, dass mindestens zwei Infrarot-Absorptionslinien des Probegases in dem definierten Spektralbereich liegen und dass die Auswerteinheit (8) dazu ausgebildet und eingerichtet ist, anhand der bei den zwei Absorptionslinien erfolgenden Lichtabsorption das Verhältnis zweier Isotope, Elemente oder Moleküle des Probegases in Echtzeit zu bestimmen.

3. Messvorrichtung (100) nach Anspruch 1, **dadurch gekennzeichnet, dass** die Messvorrichtung (100) derart ausgebildet und eingerichtet ist, dass eine zeitaufgelöste Lichterfassung durch die Detektionsvorrichtung (61) während des Durchströmens des Probegases durch die Messkammer (2) erfolgt.

4. Messvorrichtung (100) nach einem der vorangehenden Ansprüche, **gekennzeichnet durch** ein Spirometer (5), das den Volumenstrom des Probegases, das **durch** die Messkammer (2) strömt, erfasst.

5. Messvorrichtung (100) nach einem der vorangehenden Ansprüche, **gekennzeichnet durch** eine Vorkammer (4), die dazu ausgebildet ist, das Probegas auf eine bestimmte Temperatur zu erwärmen oder abzukühlen und den Wasserdampfgehalt des Probegases zu reduzieren.

6. Messvorrichtung (100) nach einem der vorangehenden Ansprüche, **dadurch gekennzeichnet, dass** die Detektionsvorrichtung (61) dazu ausgebildet und eingerichtet ist, pro Spektralbereich, in dem die Laserfrequenz variiert, mehr als 20 Messpunkte, insbesondere mehr als 100 Messpunkte, insbesondere mehr als 500 Messpunkte zu messen.

7. Messvorrichtung (100) nach einem der vorangehenden Ansprüche, **dadurch gekennzeichnet, dass** das Lasersignal gepulst ist mit einer Pulsdauer von bevorzugt weniger als 200 ns, insbesondere von weniger als 100 ns.

8. Messvorrichtung (100) nach einem der vorangehenden Ansprüche, **dadurch gekennzeichnet, dass** die Detektionsvorrichtung (61) dazu ausgebildet und eingerichtet ist, zu jedem ausgesandten Lichtpuls des Lasers (1) eine Absorptionsmessung durchzuführen.

9. Messvorrichtung (100) nach einem der vorangehenden Ansprüche, **dadurch gekennzeichnet, dass** die Detektionsvorrichtung (61) dazu ausgebildet und eingerichtet ist, mit einer Frequenz ausgelesen zu werden, die doppelt so groß ist wie die Frequenz, mit der der Laser (1) Lichtpulse aussendet.

10. Messvorrichtung (100) nach einem der vorangehenden Ansprüche, **dadurch gekennzeichnet, dass** die Messvorrichtung derart ausgestaltet ist, dass das vom Laser (1) emittierte Licht in zwei Teilstrahlen aufgeteilt wird, wobei der eine Teilstrahl durch die Messkammer (2) läuft und der andere Teilstrahl von einer Referenzdetektionsvorrichtung (62) erfasst wird, und wobei die Auswerteinheit (8) die Signale der Referenzdetektionsvorrichtung zur Normierung der Signalstärke des Lasers (1) auswertet.

11. Messvorrichtung (100) nach Anspruch 1, **dadurch gekennzeichnet, dass** die Messvorrichtung (100) dazu ausgebildet und eingerichtet ist, die Kohlendioxidkonzentration des Atemgases im Bereich zwischen 0.08% und 8% im Durchfluss in Echtzeit zu bestimmen.

12. Messvorrichtung (100) nach einem der vorangehenden Ansprüche, **dadurch gekennzeichnet, dass** die Messvorrichtung dazu ausgebildet und eingerichtet ist, die Linienbreite einer Infrarot-Absorptionslinie des Probegases in Abhängigkeit von der Gaskonzentration zu bestimmen.

13. Verfahren zur Untersuchung eines Probegases mittels Infrarot-Absorptionsspektroskopie in einer Messvorrichtung (100) gemäß einem der vorhergehenden Ansprüche, mit den Schritten:
- Durchstrahlen einer Messkammer (2) mit Licht, das von einem schmalbandigen Laser (1) emittiert wird, dessen Linienbreite kleiner oder gleich der Breite einer zu messenden Infrarot-Absorptionslinie eines in der Messkammer (2) befindlichen Probegases ist und bei unter 0,2 cm⁻¹ liegt, wobei die Laserfrequenz periodisch innerhalb eines definierten Spektralbereichs variiert wird und die Laserfrequenz und deren Variation derart gewählt werden, dass mindestens eine zu messende Infrarot-Absorptionslinie des Probegases in dem definierten Spektralbereich liegt,
- zeitaufgelöstes Erfassen des vom Laser (1) emittierten und die Messkammer (2) durchstrahlten Lichts, wobei in dem definierten Spektralbereich eine Mehrzahl von Messpunkten erfasst wird, die jeweils einer einzelnen Absorptionsmessung entsprechen, und wobei eine einzelne Absorptionsmessung innerhalb von 10⁻⁵ s oder schneller durchgeführt wird, und
- Auswerten der erfassten Signale hinsichtlich einer in der Messkammer (2) erfolgten Lichtabsorption, wobei die Lichtabsorption innerhalb des definierten Spektralbereichs frequenzaufgelöst bestimmt wird,
**dadurch gekennzeichnet,**
**dass** das Probegas das Atemgas eines Menschen oder Tieres ist, wobei das Atemgas allein durch die Atmung des Menschen oder des Tieres in der Messkammer (2) ausgetauscht wird und wobei der Atemwiderstand der Messvorrichtung (100) bei weniger als 60 mbar liegt.

14. Verfahren nach Anspruch 13, **dadurch gekennzeichnet, dass** das Verhältnis zweier Isotope, Elemente oder Moleküle des Probegases bestimmt wird, die Absorptionslinien aufweisen, die in dem definierten Spektralbereich liegen.

## Claims

1. Measurement device (100) for analyzing a sample gas by infrared absorption spectroscopy, comprising:
- a measurement chamber (2) with the sample gas to be analyzed,
- a laser (1) being arranged in relation to the measurement chamber (2) such that light being emitted from the laser radiates through the measurement chamber (2),
- a detection device (61) detecting the light being emitted from the laser (1) and radiated through the measurement chamber (2), and
- an evaluation unit (8) evaluating signals generated by the detection device (61) regarding a light absorption occurred in the measurement chamber (2), wherein
- the laser (1) is a narrowband emitting laser, the line width of which is smaller or equal to the width of an infrared absorption line to be measured of the sample gas and is less than 0.2 cm⁻¹, wherein
- the laser (1) is designed and arranged such that the laser frequency is varied periodically within a defined spectral range, wherein the laser frequency and its variation are chosen such that at least one infrared absorption line to be measured of the sample gas lies in the defined spectral range, and wherein
- the detection device (61) is designed and arranged such that it detects the light being emitted from the laser (1) and radiated through the measurement chamber (2) in such a time-resolved manner that the light absorption can be determined frequency-resolved within the defined spectral range,
**characterized**
**in that** the detection device (61) is designed and arranged to detect a plurality of measuring points in the defined spectral range which each correspond to one single absorption measurement and to carry out each single absorption measurement within 10⁻⁵ s or faster and in that the measurement device (100) is suited and can be arranged to measure the respiratory gas of a human or animal as sample gas, wherein the measurement device (100) has an open construction without valves which could hinder the flow of the sample gas and has an essentially constant cross section for the gas flowing through between gas inlet into the measurement device and gas exit out of the measurement device, such that the respiratory gas exchanges in the measurement chamber (2) only by the respiration of the human or animal, and the respiratory resistance of the measurement device (100) is less than 60 mbar.

2. Measurement (100) device according to claim 1, **characterized in that** the laser frequency and its variation are chosen such that at least two infrared absorption lines of the sample gas lie in the defined spectral range and **in that** the evaluation unit (8) is designed and arranged to determine the ratio of two isotopes, elements or molecules of the sample gas on the basis of the light absorptions occurring at the two absorption lines.

3. Measurement device (100) according to claim 1, **characterized in that** the measurement device (100) is designed and arranged such that a time-resolved light detection by the detection device (61) occurs during flowing-through of the sample gas through the measurement chamber (2).

4. Measurement device (100) according to any of the preceding claims, **characterized by** a spirometer (5) detecting the volumetric flow rate of the sample gas flowing through the measurement chamber.

5. Measurement device (100) according to any of the preceding claims, **characterized by** an ante-chamber (4) being designed to heat or cool the sample gas onto a defined temperature and to reduce the water vapor content of the sample gas.

6. Measurement device (100) according to any of the preceding claims, **characterized in that** the detection device (61) is designed and arranged to measure more than 20 measuring points, in particular more than 100 measuring points, in particular more than 500 measuring points per spectral range in which the laser frequency varies.

7. Measurement device (100) according to any of the preceding claims, **characterized in that** the laser signal is pulsed with a pulse duration of preferably less than 200 ns, in particular of less than 100 ns.

8. Measurement device (100) according to any of the preceding claims, **characterized in that** the detection device (61) is designed and arranged to carry out an absorption measurement for each emitted light pulse of the laser (1).

9. Measurement device (100) according to any of the preceding claims, **characterized in that** the detection device (61) is designed and arranged to be read out with a frequency that is twice as big as the frequency by which the laser (1) emits light pulses.

10. Measurement device (100) according to any of the preceding claims, **characterized in that** the measurement device is formed such that the light emitted from the laser (1) is divided into to two partial beams, wherein the one partial beam passes through the measurement chamber (2) and the other partial beam is detected by a reference detection device (62), and wherein the evaluation unit (8) evaluates the signals of the reference detection device for standardizing the signal intensity of the laser (1).

11. Measurement device (100) according to claim1, **characterized in that** the measurement device (100) is designed and arranged to determine the carbon dioxide concentration of the respiratory gas in the range between 0.08 % and 8 % in flow-through in real time.

12. Measurement device (100) according to any of the preceding claims, **characterized in that** the measurement device is designed and arranged to determine the line width of an infrared absorption line of the sample gas in dependence on the gas concentration.

13. Method for analyzing a sample gas by infrared absorption spectroscopy in a measurement device (100) according to any of the preceding claims, having the steps of:
- radiating a measurement chamber (2) with light being emitted from a narrowband laser (1), the line width of which is smaller or equal to the width of an infrared absorption line to be measured of a sample gas being present in the measurement chamber (2) and is less than 0.2 cm⁻¹, wherein the laser frequency is varied periodically within a defined spectral range, and the laser frequency and its variation are chosen such that at least one infrared absorption line to be measured of the sample gas lies in the defined spectral range,
- time-resolved detecting the light emitted from the laser (1) and radiated through the measurement chamber (2), wherein a plurality of measuring points were detected in the defined spectral range which each correspond to one single absorption measurement and wherein one single absorption measurement is carried out within 10⁻⁵ s or faster, and
- evaluating the detected signals regarding a light absorption occurred in the measurement chamber (2), wherein the light absorption is determined frequency-resolved within the defined spectral range,
**characterized**
**in that** the sample gas is the respiratory gas of a human or animal, wherein the respiratory gas is exchanged in the measurement chamber (2) only by the respiration of the human or animal, and wherein the respiratory resistance of the measurement device (100) is less than 60 mbar.

14. Method according to claim 13, **characterized in that** the ratio of two isotopes, elements or molecules of the sample gas is determined which have absorption lines lying within the defined spectral range.

## Revendications

1. Dispositif de mesure (100) pour l'analyse d'un échantillon gazeux par spectroscopie d'absorption infrarouge, présentant :
- une chambre de mesure (2) contenant l'échantillon gazeux à analyser,
- un laser (1) qui est disposé, par rapport à la chambre de mesure (2), de telle sorte que la lumière émise par le laser traverse la chambre de mesure (2),
- un dispositif de détection (61), qui détecte la lumière émise par le laser (1) et ayant traversé la chambre de mesure (2), et
- une unité d'évaluation (8), qui évalue, pour ce qui est d'une absorption de la lumière réalisée dans la chambre de mesure (2), les signaux produits par le dispositif de détection (61),
- le laser (1) étant un laser émettant en bande étroite, dont la largeur de raie est inférieure ou égale à la largeur d'une raie d'absorption infrarouge à mesurer de l'échantillon gazeux, et est inférieure à 0,2 cm⁻¹,
- le laser (1) étant configuré et monté de telle sorte que la fréquence du laser varie périodiquement à l'intérieur d'un domaine spectral défini, la fréquence du laser et sa variation étant choisies de façon qu'au moins une raie d'absorption infrarouge à mesurer de l'échantillon gazeux se trouve dans le domaine spectral défini, et
- le dispositif de détection (61) étant configuré et monté de façon à résoudre en temps la lumière émise par le laser (1) et ayant traversé la chambre de mesure (2), de telle sorte que l'absorption de la lumière puisse être déterminée avec résolution en fréquence à l'intérieur du domaine spectral défini,
**caractérisé en ce que** le dispositif de détection (61) est configuré et monté de façon à détecter dans le domaine spectral défini une pluralité de points de mesure, dont chacun correspond à une mesure d'absorption individuelle, et à effectuer chaque mesure d'absorption individuelle en moins de 10⁻⁵ s, ou plus vite, et **en ce que** le dispositif de mesure (100) est apte et peut être monté de façon à mesurer en tant qu'échantillon gazeux le gaz respiratoire d'un homme ou d'un animal, le dispositif de mesure (100) présentant une structure ouverte sans vannes qui pourraient empêcher l'écoulement de l'échantillon gazeux, ainsi qu'une section transversale constante pour le gaz devant s'écouler entre l'entrée du gaz dans le dispositif de mesure et la sortie du gaz du dispositif de mesure, de telle sorte que le gaz respiratoire soit remplacé uniquement par la respiration de l'homme et/ou de l'animal dans la chambre de mesure (2), et que la résistance respiratoire du dispositif de mesure (100) soit inférieure à 60 mbar.

2. Dispositif de mesure (100) selon la revendication 1, **caractérisé en ce que** la fréquence du laser et sa variation sont choisies de façon qu'au moins deux raies d'absorption infrarouge de l'échantillon gazeux se trouvent dans le domaine spectral défini, et que l'unité d'évaluation (8) soit configurée et montée de façon à déterminer en temps réel, à l'aide de l'absorption de la lumière qui a lieu aux deux raies d'absorption, le rapport entre deux isotopes, éléments ou molécules de l'échantillon gazeux.

3. Dispositif de mesure (100) selon la revendication 1, **caractérisé en ce que** le dispositif de mesure (100) est configuré et monté de telle sorte qu'une détermination de la lumière avec résolution en temps soit réalisée par le dispositif de détection (61) pendant la traversée de la chambre de mesure (2) par l'échantillon gazeux.

4. Dispositif de mesure (100) selon l'une des revendications précédentes, **caractérisé par** un spiromètre (5), qui détermine le débit volumique de l'échantillon gazeux qui traverse la chambre de mesure (2).

5. Dispositif de mesure (100) selon l'une des revendications précédentes, **caractérisé par** une préchambre (4), qui est configurée de façon à chauffer ou refroidir l'échantillon gazeux à une certaine température, et à réduire la teneur de l'échantillon gazeux en vapeur d'eau.

6. Dispositif de mesure (100) selon l'une des revendications précédentes, **caractérisé en ce que** le dispositif de détection (61) est configuré et monté de façon à mesurer, par domaine spectral dans lequel varie la fréquence du laser, plus de 20 points de mesure, en particulier plus de 100 points de mesure, en particulier plus de 500 points de mesure.

7. Dispositif de mesure (100) selon l'une des revendications précédentes, **caractérisé en ce que** le signal laser est pulsé avec une durée d'impulsion de préférence inférieure à 200 ns, en particulier inférieure à 100 ns.

8. Dispositif de mesure (100) selon l'une des revendications précédentes, **caractérisé en ce que** le dispositif de détection (61) est configuré et monté de façon à effectuer une mesure de l'absorption pour chaque impulsion lumineuse émise du laser (1).

9. Dispositif de mesure (100) selon l'une des revendications précédentes, **caractérisé en ce que** le dispositif de détection (61) est configuré et monté de façon à être lu à une fréquence qui est le double de la fréquence à laquelle le laser (1) émet des impulsions lumineuses.

10. Dispositif de mesure (100) selon l'une des revendications précédentes, **caractérisé en ce que** le dispositif de mesure est équipé de telle sorte que la lumière émise par le laser (1) soit divisée en deux faisceaux partiels, l'un des faisceaux partiels traversant la chambre de mesure (2) et l'autre faisceau partiel étant détecté par un dispositif de détection de référence (62), et l'unité d'évaluation (8) évaluant les signaux du dispositif de détection de référence pour normer l'intensité du signal du laser (1).

11. Dispositif de mesure (100) selon la revendication 1, **caractérisé en ce que** le dispositif de mesure (100) est configuré et monté de façon à déterminer pendant l'écoulement en temps réel la concentration du dioxyde de carbone dans le gaz respiratoire dans la plage de 0,08 % à 8 %.

12. Dispositif de mesure (100) selon l'une des revendications précédentes, **caractérisé en ce que** le dispositif de mesure est configuré et monté de façon à déterminer la largeur de raie d'une raie d'absorption infrarouge de l'échantillon gazeux en fonction de la concentration du gaz.

13. Procédé pour analyser un échantillon gazeux par spectroscopie d'absorption infrarouge dans un dispositif de mesure (100) selon l'une des revendications précédentes, comportant les étapes suivantes :
- irradiation d'une chambre de mesure (2) avec une lumière qui est émise par un laser à bande étroite (1), dont la largeur de raie est inférieure ou égale à la largeur d'une raie d'absorption infrarouge à mesurer d'un échantillon gazeux se trouvant dans la chambre de mesure (2) et est inférieure à 0,2 cm⁻¹, la fréquence du laser variant périodiquement à l'intérieur d'un domaine spectral défini, et la fréquence laser et sa variation étant choisies de façon qu'au moins une raie d'absorption infrarouge à mesurer de l'échantillon gazeux se trouve dans le domaine spectral défini,
- détermination, avec résolution en temps, de la lumière émise par le laser (1) et ayant traversé la chambre de mesure (2), dans le domaine spectral défini une pluralité de points de mesure étant déterminée, dont chacun correspond à une mesure d'absorption individuelle, et une mesure d'absorption individuelle étant effectuée en moins de 10⁻⁵ s, ou plus vite, et
- évaluation des signaux détectés, pour ce qui est d'une absorption de la lumière effectuée dans la chambre de mesure (2), l'absorption de la lumière étant déterminée avec une résolution en fréquence à l'intérieur du domaine spectral défini,
**caractérisé en ce que** l'échantillon gazeux est le gaz respiratoire d'un homme ou d'un animal, le gaz respiratoire étant remplacé uniquement par la respiration de l'homme ou de l'animal dans la chambre de mesure (2), et la résistance respiratoire du dispositif de mesure (100) étant inférieure à 60 mbar.

14. Procédé selon la revendication 13, **caractérisé en ce qu'**on détermine le rapport entre deux isotopes, éléments ou molécules de l'échantillon gazeux, qui présentent des raies d'absorption qui se trouvent dans le domaine spectral défini.
